# EUROPEAN PATENT SPECIFICATION

(11) **EP 3 971 604 B1**
(45) Date of publication and mention of the grant of the patent: **26.04.2023**
(21) Application number: 20196769.2
(22) Date of filing: 17.09.2020
(51) Int. Cl.: G01S 7/35, G01S 7/41, G01S 13/34, G01S 13/42, G01S 13/86, G01S 13/87, G01S 13/88, A61F 2/58, B25J 15/00, B25J 19/02, A61F 2/70, A61F 2/68

(54) **PROSTHETIC HAND**
PROTHESENHAND
PROTHÈSE DE MAIN

(43) Date of publication of application: 23.03.2022
(73) Proprietor: Fraunhofer-Gesellschaft zur Förderung der angewandten Forschung e.V., 80686 München (DE)
(72) Inventor: AZIZ, Fady, 70569 Stuttgart (DE)
(74) Representative: Pfenning, Meinig & Partner mbB

(56) References cited:
- GB-A- 2 574 596
- US-A1- 2009 285 664
- US-A1- 2019 237 354
- SHERIF ABDULATIF ET AL: "Stairs Detection for Enhancing Wheelchair Capabilities Based on Radar Sensors", ARXIV.ORG, CORNELL UNIVERSITY LIBRARY, 201 OLIN LIBRARY CORNELL UNIVERSITY ITHACA, NY 14853, 25 November 2017 (2017-11-25), XP081299601, DOI: 10.1109/GCCE.2017.8229270

## Description

The invention relates to a prosthetic hand having a first radar module attached the prosthetic hand at a wrist joint of the prosthetic hand, wherein the first radar module is configured to receive a first radar signal reflected by a target object and generate a first property signal based on the received first radar signal, and the prosthetic hand further comprises a second radar module attached to a finger of the prosthetic hand, wherein the second radar module is configured to receive a second radar signal reflected by the target object and generate a second property signal based on the received second radar signal. The invention furthermore concerns a method to be carried out with said prosthetic hand, wherein in a transport phase the first radar module receives the first radar signal reflected by the target object and the wrist moves towards the target object, and in a grip phase the second radar module receives the second radar signal reflected by the target object based on which a material of the target object is determined and at least one finger of the prosthetic hand forms to grip the target object.

Grasping and gripping, which are used as synonyms here, is considered one of the main activities for prosthetic hand users. An important aspect for the grasping activity is identifying properties of a target object to be grasped as for example the object dimensions. Furthermore, other aspects as for example a material of the target object, a position and/or an orientation of the target object can be important.

GB 2 574 596 A discloses a prothetic device comprising means for engaging a wearer, at least a palm section carrying at least one digit operated by at least one actuator and sensor means mounted to the palm section of the device and arranged to provide sensor signals representing objects in the vicinity of the device. The device further comprises control means coupled to the sensor means and to at least one actuator and configured to drive the at least one actuator in response to sensor signals.

It is therefore the object of the present invention to provide a prosthetic hand as well as a method for operating a prosthetic hand, which improves the grasping procedure.

The object is solved by the prosthetic hand according to claim 1 and the method for operating a prosthetic hand according to claim 8. The corresponding dependent claims describe advantageous embodiments of the prosthetic hand according to claim 1 and the method for operating a prosthetic hand according to claim 8.

The invention concerns a prosthetic hand having a wrist joint as well as at least one finger. Preferably, the prosthetic hand can be constructed like a human hand wherein the wrist joint can be attached to an end of a forearm as well as a palm to which at least one, preferably multiple, in particular five fingers are attached. Preferably the fingers are attached to the palm via further joints. The at least one finger is constructed such that it can grasp a target object.

According to the invention, a first radar module is attached to the prosthetic hand at the wrist joint. The radar module can be attached at or in the vicinity of that place where a possible forearm ends and the palm begins, but it is also possible that the first radar module is attached to the possible forearm itself or to the palm, preferably adjacent to the wrist joint. The term "adjacent" can for example describe a distance to the wrist joint smaller than 10 cm, preferably smaller than 5 cm, in particular preferred smaller than 1 cm, the distance being measured from the pivot point of the wrist joint to the closest edge of the radar module.

The first radar module is configured to receive a first radar signal reflected by a target object and generate a first property signal based on the received radar signal. A property signal is a signal which allows determining properties of the target object from this signal. From the first property signal preferably geometric properties can be determined, as for example object dimensions. The determination can for example be a calculation or a determination using a neural network. Preferable the property of the target object determined from the first property signal can be one or more of the following properties: Shape of the target object, a size of the target object, a height of the target object, a width of the target object and/or sharp edges of the target object.

The radar module can have a certain field of vision. In this case the field of vision is preferably directed such that the target object is within the field of vision when the prosthetic hand approaches the target object in order to grasp the target object. Such a field of vision can preferably be directed in the direction of the at least one finger.

The prosthetic hand according to the invention furthermore comprises a second radar module which is attached to at least one of the at least one fingers, wherein the radar module is configured receive a second radar signal reflected by the target object and generate a second property signal based on the received second radar signal. The second property signal is used to detect a material of the target object. The determination of the material can for example be embodied as a classification of the material.

The prosthetic hand according to the invention furthermore comprises a processing unit which is configured to receive the property signals from the first radar module and the second radar module and to calculate at least one property of the target object based on the property signals received from the first radar module and the second radar module. Such properties can in particular be those which are mentioned above.

The first and/or the second radar modules preferably comprise at least a radar receiver to receive the first and second radar signal respectively. Advantageously, the first and/or second radar module also comprises a radar transmitter which transmits the signal which is reflected as the first or second reflected signal, respectively.

In a preferred embodiment of the invention the first radar module and/or the second radar module can be a frequency modulated continuous wave, FMCW, radar. An FMCW radar is in particular preferred for environmental sensing in which the respective radar module identifies the position of the target.

Preferably, the first and the second radar modules can have different beam forming capabilities. Preferably one radar module, in particular preferred the first radar module, can be a multiple input - multiple output, MIMO, module, which in advantageously has scanning capability. Such a MIMO module can for example be employed to detect an object orientation and/or object dimensions.

In an advantageous embodiment the prosthetic hand can further include an inertial measurement unit, IMU. The IMU can preferably be arranged at or in the wrist joint or the palm. It allows detecting and if necessary correcting a tilt angle of the prosthetic hand or the palm, and thereby ensuring accurate target positioning.

The use of radar modules is particularly advantageous if the prosthetic hand is to operate under harsh environmental conditions such as for example darkness, smoke, dust and/or different temperature conditions.

Regarding the second radar module, it is in particularly preferred if this radar module generates the second property signal such that a material of the target object can be determined based on this second property signal. For example the radar module can be capable of detecting whether the target object comprise electronics, glass, or wood, but it can also detect whether it is a phone, a mug, a tablet or any other object.

The present invention also concerns a method for operating a prosthetic hand, wherein the prosthetic hand is constructed as described above.

The method according to the invention comprises a transport phase in which the first radar module receives a first radar signal reflected by the target object and generates a first property signal based on the first radar signal. Based on the first property signal at least one property of the target object, preferably a property as for example a primary shape of the target object, a size of the target object, a height of the target object, a width of the target object and/or sharp edges of the target object, is determined. The analysis of the first property signal provide by the first radar module can for example reveal a primary shape of the object and/or dimensions as for example height and width of the object. It can also reveal geometric characteristics of the object as for example sharp edges.

Preferably the prosthetic hand moves towards the object in the transport phase. Advantageously, the transport phase can be defined as that time during which the prosthetic hand or the wrist moves towards the object, in particular before grasping the object. Thus, the transport phase can for example start with begin of the movement and end with begin of the grip phase.

The method according to the invention furthermore comprises a grip phase or gripping phase, in which the second radar module receives a second radar signal reflected by the target object and generates a second property signal based on the second radar signal. From the second property signal a material of the target object is detected. In the grip phase the at least one finger forms to grip the target object. As "detecting a material" should also be understood that the second radar module captures raw data reflected back from the target object which raw data are then used to determine the material of the target object. For example, the second radar module can pass the detected raw data to a control panel where a non-contact object classification can be performed.

In a preferred embodiment of the method according to the invention it can be detected that the prosthetic hand is in the transport mode by detecting an angular motion pattern of the prosthetic hand, for example by using the IMU. The transport phase, during which the prosthetic hand moves towards the target object, has a specific angular motion pattern. If this pattern is detected it can be concluded that the prosthetic hand is in the transport phase.

In a preferred embodiment the first radar module can perform a scanning process in which a two-dimensional map of the target object in a plane intersecting with the target object is captured. In particular, the first radar module can perform real time beam forming and capture the two-dimensional map for sagittal plane. It is then preferably possible that the current tilt angle of the wrist joint or the hand is acquired, for example by said optional MIMO, and the two-dimensional map can be corrected based on the tilt angle. Said tilt angle can for example be defined as the angle between a plane in which the palm extends or a plane in which the palm and at least one finger extends or a plane in which the fingers extend if they are in a straight shape and for example a horizontal plane. Of course other possible definitions of the tilt angle are also applicable.

In a preferred embodiment a primary shape of the target object, a size of the target object, a height of the target object, a width of the target object, sharp edges of the target object, position of the target object and/or an orientation of the target object can be estimated by adaptive filtering of the results of the scanning process such as particle filtering. 'Abdulatif, Sherif, et al. "Stairs detection for enhancing wheelchair capabilities based on radar sensors." 2017 IEEE 6th Global Conference on Consumer Electronics (GCCE). IEEE, 2017.' 'Guo, Wenyan, Chongzhao Han, and Ming Lei. "Improved unscented particle filter for nonlinear Bayesian estimation." 2007 10th International Conference on Information Fusion. IEEE, 2007.'

Advantageously, a material of the target object can be detected based on changes in a detection signal recorded by the second radar module. This, for example, can be done by applying a predictive model machine learning technique. For example a random forest technique as described in the reference "RadarCat: Radar Categorization for Input & Interaction", Hui-Shyong Yeo, Gergely Flamich, Patrick Schrempf, David Harris-Birtill and Aaron Quigley, SACHI, School of Computer Science University of St Andrews, Scotland, UK, published in the repository of the University of St. Andrews.

In principle, any machine learning technique from the family of complex trees techniques can be employed. These techniques show better performance when trained with as much features as possible from the signals. One way for boosting the used features is through formulating hand manufactured features as for example "average" and "standard deviation".

For this technique the second radar module can, for example, be a monostatic radar device comprising multiple antennas, for example two transmitters and four receivers, which provide simultaneous operation of up to N-channels, using frequency modulated continuous wave, operating in the mm-wave band 30 - 300 GHz. As the application is considered for health care, the radar can be operating with the range of 40 - 70 GHz, for example with a center frequency of 60 GHz.

In a preferred embodiment of the invention disturbances between the first and/or second sensor on the one hand side and the target object on the other hand side can be compensated by employing machine learning techniques and/or a deep learning techniques, preferable by using a generative adversarial network. To perform this compensation, a network can be trained with paired data, each pair containing contact data and non-contact data. Here, the contact data are the clean data showing the signal structure for each material when the object is in direct contact with the radar module. The non-contact data of each pair show the structure of the raw data when the materials are not in contact with the radar module in different scenarios. After such kind of training the network will be able to remove any environmental effects which were available in the training data. Details of how this training and use of generative adversarial networks can be found for example in the reference "An Adversarial Super-Resolution Remedy for Radar Design Trade-offs", Karim Armanious, Sherif Abdulatif, Fady Aziz, Urs Schneider and Bin Yang, in arXiv:1903.01392v2, 20 Jun 2019.

A further description of this kind of compensation can also be found in the reference "Towards Adversarial Denoising of Radar Micro-Doppler Signatures", Sherif Abdulatif, Karim Armanious, Fady Aziz, Urs Schneider, Bin Yang, in arXiv:1811.04678v3, 7 Aug 2019.

In the following the invention is described by wave examples with reference to figures. The features shown in the examples can also be combined among different examples and can be realized independently from the specific example.
- Fig. 1: shows an example of a prosthetic hand according to the invention,
- Fig. 2: shows a situation in a transport phase,
- Fig. 3: shows a situation in a grip phase,
- Fig. 4: shows an example for a course of the method according to the invention,
- Fig. 5: shows received radar signals for different kinds of objects,
- Fig. 6: shows received radar signals in contact with the target object and not in contact with the target object,
- Fig. 7: shows a comparison between received radar signals of a phone in contact and not in contact,
- Fig. 8: shows an example of a method for material classification,
- Fig. 9: shows an example interaction between possible units of the prosthetic hand of the invention, and
- Fig. 10: shows an algorithm hardware structure which can be used to implement the invention.

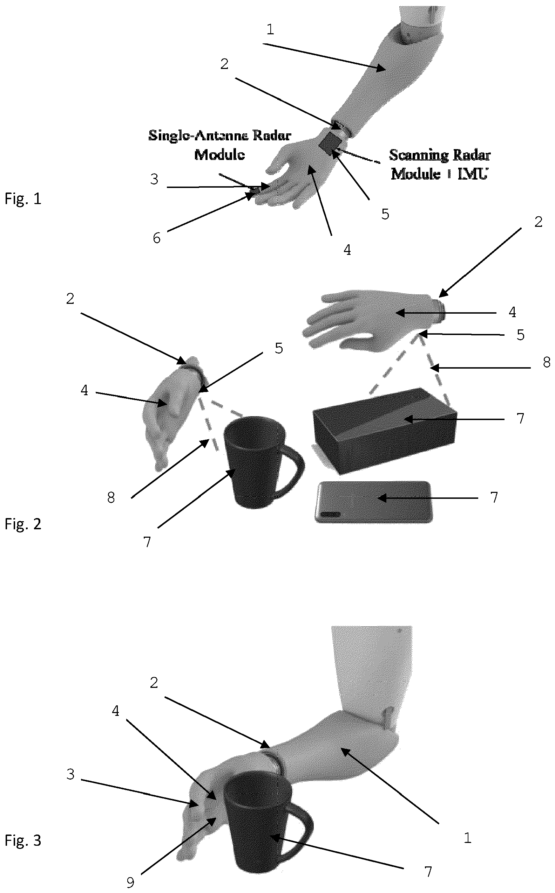

Fig. 1 shows a prosthetic hand according to the invention. The prosthetic hand in Fig. 1 is attached to a prosthetic forearm 1 via a wrist joint 2, which shall be regarded as part of the prosthetic hand. The prosthetic hand in the example of Fig. 1 has a palm 4 as well as five fingers. The prosthetic hand comprises a first radar module 5 which is attached to the prosthetic hand at the wrist joint 2 as well as a second radar module 6 which is attached to one of the fingers 3. The first radar module 5 is configured to receive a first radar signal reflected by a target object 7 and to generate a first property signal based on a received first radar signal. The radar module 5 can also have a radar transmitter to transmit a radar signal which is reflected as the first radar signal.

The second radar module is configured to receive a second radar signal reflected by the target object and to generate the second property signal based on the received second radar signal. Also the second radar module can have a transmitter to transmit a radar signal which is reflected as the second radar signal.

The prosthetic hand furthermore comprises a processing unit which is configured to receive the first and second property signals from the first radar module and the second radar module. The processing unit can then calculate at least one property of the target object based on the first property signal and furthermore can calculate a material of the target object based on the second property signal. It should be noted that the processing unit is not shown in the figures. It can be integrated with the prosthetic hand or can be a separate element which can be connected to the prosthetic hand by a suitable connection as for example a cable connection or a radio connection.

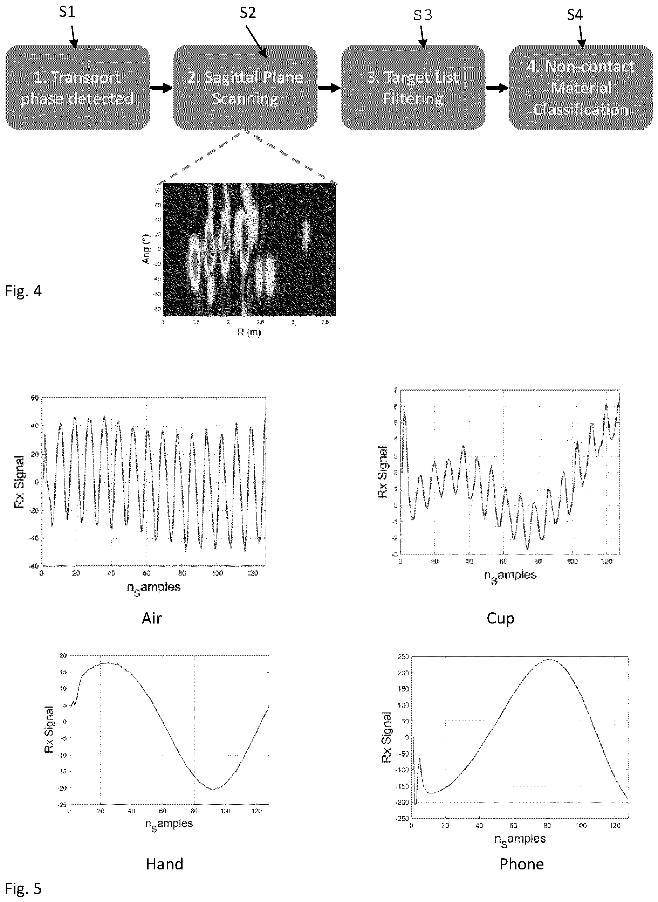

In Fig. 1 the first radar module 5 is attached to the prosthetic hand at the wrist joint 2. To be more specific, in Fig. 5 the first radar module 5 is attached to a part of the prosthetic hand or the palm 4 in which the wrist joint is inserted. Thus, the first radar module is attached to the prosthetic hand at the first wrist joint.

The finger 3 can bend in the direction of the first radar module and thereby grip a target object.

Fig. 2 shows two examples of a prosthetic hand according to the invention relative to target objects 7 in a transport phase in which the prosthetic hand moves towards the corresponding target object 7. The first radar module in Fig. 2 transmits radar signals 8 towards the target object 7 and receives the first radar signal reflected by the target object 7. The target object can for example be a box, a phone, or a cup.

Fig. 3 shows the prosthetic hand in a gripping phase in which the target object 7 is gripped. In this grip phase the second radar module transmits a second radar signal 9 towards the target object 7 and receives the reflected signal as second radar signal. From this second radar signal the second radar module generates a second property signal from which the processing unit can then determine the material of the target object.

Fig. 4 shows an example of a method according to the present invention. In this method a prosthetic hand as for example described in Figs. 1 to 3 is operated. The prosthetic hand in a so-called transport phase approaches the target object 7. The transport phase can be detected in a step S1 for example by detecting the movement of the prosthetic hand. This can be done for example with an IMU.

During the transport phase the first radar module receives a first radar signal reflected by the target object. In the specific example of Fig. 4 the first radar module performs sagittal plane scanning in step S2 resulting in a first radar signal as shown below step S2 in Fig. 4. On the perpendicular axis the angle is shown and in the horizontal axis the distance from the radar module is shown. The MIMO radar can be featured with a scanning antenna that is capable of scanning in both horizontal and vertical directions. Accordingly, the angle information may indicate both the height and the width of the object.

In step S3 target list filtering is performed. A 2D-scan of the sagittal plane is performed for example through multi-dimensional FFT. After applying the adaptive filtering techniques such as the CFAR and the particle filter, the output will be a target list formulated in the 2D or 3D space. Each component in the target list is referring to a detected target with indices in the X-Y-Z plane referring to the detected object in the sagittal plane.

The method shown in Fig. 4 furthermore comprises determination of a material of the target object. The material can be determined from a second property signal generated by the second radar module based on the second radar signal.

Fig. 5 shows different received radar signals as received by the second radar module. Thus, Fig. 5 shows second radar signals in above mentioned sense. The upper left diagram shows the radar signal as reflected by air, the upper right diagram shows the radar signal as reflected by a cub, the lower left diagram shows a radar signal as reflected by a hand and the lower right diagram shows a radar signal as reflected by a phone. The vertical axis represents the reflected energy from each material while the horizontal axis represents the time axis of each radar chirp.

It can be seen in Fig. 5 that different materials lead to different and characteristic radar signals. Thus, by detecting the radar signal with the second radar module, it is possible to conclude the material on which the radar signal was reflected. This can for example be achieved by classifying the received second radar signals using a neural network.

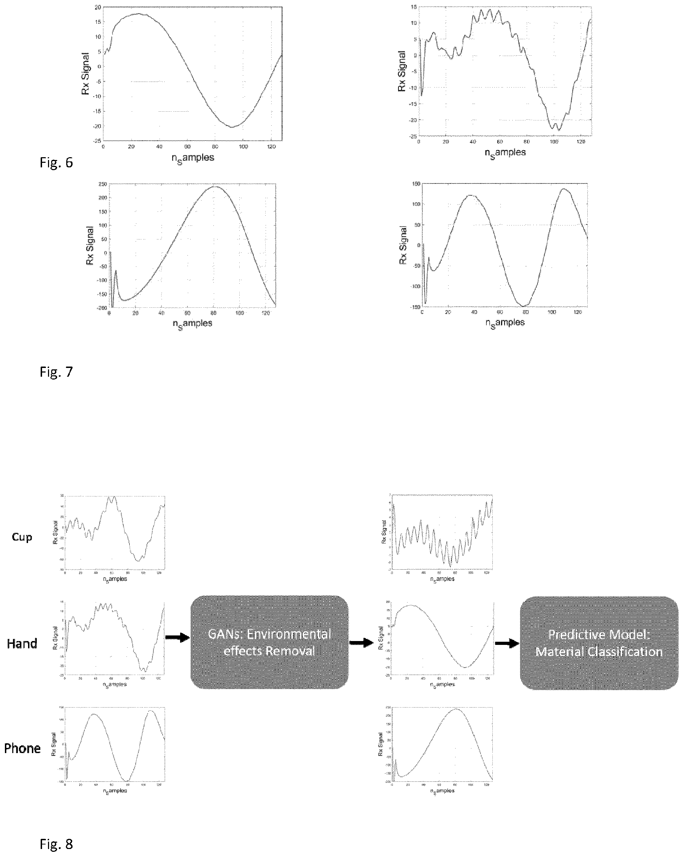

The process shown in Fig. 4 can for example start when the prosthetic hand is in the transport mode which could be detected by analyzing the angular motion activity of the prosthetic hand. Afterwards the signal processing chain can for example start in which the scanning radar, that is the first radar module, starts a real time beam forming process in which a 2D-map is captured for a sagittal plane. Then, the tilt angle can be acquired from for example an integrated IMU for map correction, and adaptive filtering can be done on these maps to extract the target's dimensional characteristics, position and orientation. When the hand starts its re-shaping for the grip phase, the second radar module can acquire the raw data reflected by the target. A direct contact between the radar and the object leads to an observable change in the signal characteristics in terms of phase and magnitude as shown in Figs. 6 and 7. A machine learning technique as for example Random Forest can be used as a predictive model for differentiating between the different materials of the target object.

Figs. 6 and 7 show a comparison between direct contact of the second radar module with the target object and non-contact cases in which the second radar module is not in direct contact with the target object. In Figs. 6 and 7 the left diagrams show the contact case and the right diagrams show the non-contact cases.

When applying a distance between the object and the sensor, environmental effects as for example air propagation or clutters will change the characteristics of the captured data as shown in Figs. 6 and 7. Deep learning techniques can be used for preserving the characteristics of a captured radar data and environmental effects can be removed. One technique which can be employed for this is for example the use of generative adversarial networks (GANs). These can be used for the task of data resolving. This technique is based on learning the characteristic distribution of the input data and achieving data generation with the same characteristics at the input data. Accordingly the technique can be used for environmental effects removal and material classification can be achieved, while the object is not in direct contact with the radar sensor.

Fig. 8 shows a possible course of such a classification task. The different diagrams on the left hand side in Fig. 8 correspond to received radar signals. The uppermost diagram represents a signal received from a cup, the middle diagram represents a signal received from a hand and the lower diagram represent a signal received from a phone. These radar signals enter a GAN which removes environmental effects. The GAN outputs radar signals in which the environmental effects have been removed. These radar signals can now be entered into a predictive model which allows material classification.

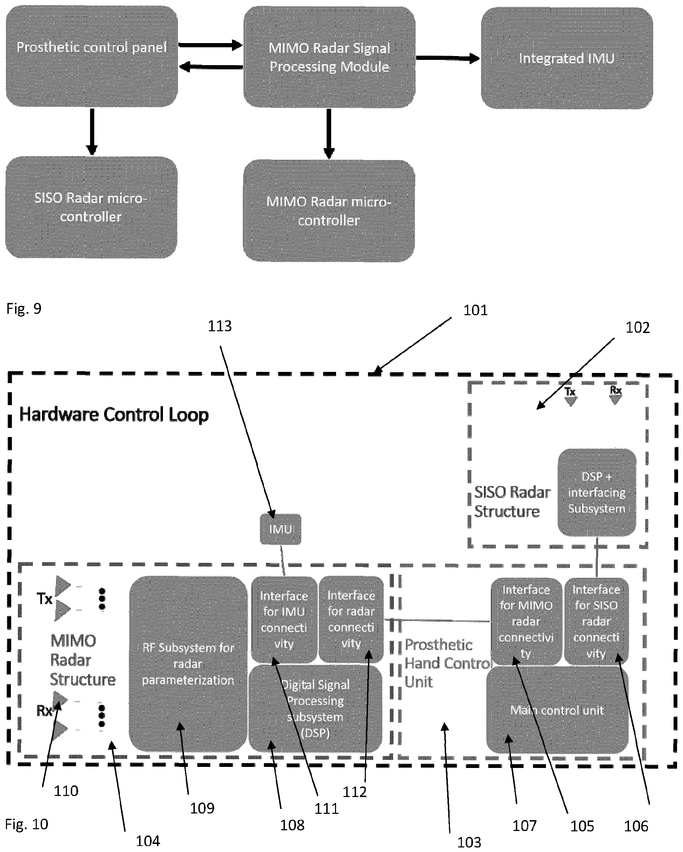

Fig. 9 shows a possible hardware control loop for the present invention. As shown in Fig. 9, a MIMO radar can include a specific micro-controller acting as the signal processing module, which for example can also carry out target list formulation and dimensions extraction.

Here a prosthetic control panel communicates with a SISO radar microcontroller and a MIMO radar signal processing module. The MIMO radar signal processing module communicates with a MIMO radar microcontroller and an integrated IMU.

The processing module in the example shown in Fig. 9 is in contact with an integrated IMU for angle correction and accurate target localization. The module can reply to the control unit with the availability of the targets including estimates about dimensions and localizations. After selecting a target of interest for grasping, that is a target object, the control unit can start a finger-attached (SISO) module, which can then provide raw data required for classification to the control unit. Machine and/or deep learning techniques can be optimized to be embedded-coded inside the control unit in addition to the control loop for contacting the first and second radar module.

Fig. 10 shows an algorithm hardware structure 101 which can be used to implement the invention. A prosthetic hand control unit 103 can be featured with three or more subsystems 105, 106, 107 and each subsystem can indicate one or two micro-controllers. The first subsystem of the prosthetic hand control unit 103 can be a main control unit 107 where a main algorithm including any learning technique can be implemented. This main control unit 107 can also be responsible for sending orders to the interfacing subsystems that are responsible for offering the required connectivity with both the SISO and the MIMO radar modules. The second subsystem 105 can responsible for offering the required connectivity with the MIMO radar module 104. When the prosthetic hand is in the transport phase, it can send an order of operation through the interfacing subsystem to the MIMO radar to start the sagittal plane scanning. After the scanning is done successfully, a primary formulated decision about both the shape and location of the target of interest can be parsed and sent to the prosthetic hand control unit 103 through the interfacing subsystem 105. Then, the main control unit 107 can send an order of operation through the third subsystem 106 which can be responsible for offering the required interfacing with a SISO radar module 102. The SISO radar module 102 can start transmission and can send back the acquired raw data to the prosthetic hand control unit 103 to be classified by the learning algorithm which is embedded implemented.

The MIMO radar can for example be featured with four or more subsystems. Each subsystem can indicate one or two micro-controllers. The first subsystem can be a digital signal processing (DSP) 108 which can be the main controlling subsystem where the main algorithm including the adaptive filtering, any required FFT to formulate the target list can be implemented. It can also be responsible for sending the other interfaces orders and parameters in order to be able to fulfill their tasks. The second subsystem can be a RF subsystem 109 which can be considered for parameterizing the transmitting and receiving antennas 110 and also adjusting the bandwidth, the chirp duration etc. The RF subsystem 109 can do its task through receiving certain orders from the DSP subsystem 108. The third 111 and fourth 112 subsystems can be interfacing subsystems responsible for offering connectivity with both the prosthetic hand control unit 103 and the IMU 113. For the SISO radar module 102, usually one subsystem can be enough for both interfacing and any required algorithm implementation if available.

## Claims

1. Prosthetic hand comprising,
a wrist joint (2),
at least one finger (3),
a first radar module (5) attached to the prosthetic hand at the wrist joint (2), the first radar module (5) being configured to receive a first radar signal reflected by a target object (7) and generate a first property signal based on the received first radar signal,
a second radar module (6) attached to the finger, the second radar module (6) being configured to receive a second radar signal reflected by the target object (7) and generate a second property signal based on the received second radar signal,
a processing unit configured to receive the first and second property signals and determine at least one property of the target object (7) based on the first property signal and determine a material of the target object (7) based on the second property signal.

2. Prosthetic hand according to the previous claim, wherein the processing unit is configured to detect based on the first property signal a primary shape of the target object (7), a size of the target object (7), a height of the target object (7), a width of the target object (7) and/or sharp edges of the target object (7).

3. Prosthetic hand according to any of the preceding claims, wherein the prosthetic hand further comprises at least on sensor, preferably an inertial measurement unit, configured to measure a tilt angle and/or an angular velocity of the prosthetic hand.

4. Prosthetic hand according to any of the preceding claims wherein the first radar module (5) and/or the second radar module (6) is a Frequency Modulated Continuous Wave, FMCW, radar.

5. Prosthetic hand according to any of the preceding claims wherein the first radar module (5) is a Multiple Input, Multiple Output, MIMO, radar.

6. Prosthetic hand according to any of the preceding claims, wherein the first radar module (5), the second radar module (6) and the processing unit are interconnected in a hardware control loop in which the first radar module (5) is part of a MIMO radar module (104), the second radar module (6) is part of a SISO radar module (102) and the processing unit is part of a prosthetic hand control unit,
wherein the MIMO radar module (104), the SISO radar module (102) and the prosthetic hand control unit are each implemented in at least one micro controller.

7. Prosthetic hand according to the preceding claim, wherein the MIMO radar module (104) comprises a Digital Signal Processing unit (DSP) (108), configured to carry out an adaptive filtering process, a Fast Fourier transform and/or configured to control other units of the MIMO radar module (104), and/or a radio frequency subunit, configured to parametrize transmitting and receiving antennas of the MIMO radar module (104) and/or adjust a bandwidth and/or chirp duration.

8. Method for operating a prosthetic hand, the prosthetic hand being an prosthetic hand according to one of the preceding claims, wherein
in a transport phase the first radar module (5) receives the first radar signal reflected by the target object (7) and the wrist (2) moves toward the target object (7) and
in a grip phase the second radar module (6) receives the second radar signal reflected by the target object (7) and the at least one finger (3) forms to grip the target object (7),
wherein at least one property is determined based on the first property signal generated by the first radar module (5) based on the received first radar signal and
wherein a material of the target object (7) is determined from the second property signal generated by the second radar module (6) based on the second radar signal.

9. Method according to the preceding claim, wherein during the transport phase a primary shape of the target object (7), a size of the target object (7), a height of the target object (7), a width of the target object (7) and/or sharp edges of the target object (7) are detected.

10. Method according to one of claims 8 or 9, wherein it is detected that the prosthetic hand is in the transport phase from an angular motion pattern of the prosthetic hand.

11. Method according to one of claims 8 to 10, wherein the first radar module (5) performs a scanning process in which a two-dimensional map of the target object (7) in a plane intersecting with the target object (7) is captured.

12. Method according to the preceding claim wherein a tilt angle is acquired, wherein based on the tilt angle the two-dimensional map is corrected.

13. Method according to one of the two preceding claims, wherein a primary shape of the target object (7), a size of the target object (7), a height of the target object (7), a width of the target object (7), sharp edges of the target object (7), a position of the target object (7) and/or an orientation of the target object (7) is calculated by adaptive filtering of the results of the scanning process.

14. Method according to one of claims 8 to 12, wherein a material of the target object (7) is detected based on changes in the second radar signal recorded by the second radar module (6).

15. Method according to one of claims 8 to 13, wherein disturbances between the first (5) and/or second radar module (6) and the target object (7) are compensated by employing a machine learning technique and/or a deep learning technique, preferably by using a Generative Adversarial Network.

## Patentansprüche

1. Prothesenhand, umfassend:
ein Handgelenk (2),
zumindest einen Finger (3),
ein erstes Radarmodul (5), das an der Prothesenhand an dem Handgelenk (2) angebracht ist, wobei das erste Radarmodul (5) eingerichtet ist, ein erstes Radarsignal, das von einem Zielobjekt (7) reflektiert wird, zu empfangen, und ein erstes Eigenschaftssignal auf der Grundlage des empfangenen ersten Radarsignals zu erzeugen,
ein zweites Radarmodul (6), das an dem Finger angebracht ist, wobei das zweite Radarmodul (6) eingerichtet ist, ein zweites Radarsignal zu empfangen, das von dem Zielobjekt (7) reflektiert wird, und ein zweites Eigenschaftssignal auf der Grundlage des empfangenen zweiten Radarsignals zu erzeugen,
eine Verarbeitungseinheit, die eingerichtet ist, das erste und zweite Eigenschaftssignal zu empfangen, und zumindest eine Eigenschaft des Zielobjekts (7) auf der Grundlage des ersten Eigenschaftssignals zu bestimmen und ein Material des Zielobjekts (7) auf der Grundlage des zweiten Eigenschaftssignals zu bestimmen.

2. Prothesenhand nach dem vorangehenden Anspruch, wobei die Verarbeitungseinheit eingerichtet ist, auf der Grundlage des ersten Eigenschaftssignals eine primäre Form des Zielobjekts (7), eine Größe des Zielobjekts (7), eine Höhe des Zielobjekts (7), eine Breite des Zielobjekts (7) und/oder scharfe Kanten des Zielobjekts (7) zu erfassen.

3. Prothesenhand nach einem der vorangehenden Ansprüche, wobei die Prothesenhand ferner mindestens einen Sensor, vorzugsweise eine inertiale Messeinheit, umfasst, der eingerichtet ist, einen Kippwinkel und/oder eine Winkelgeschwindigkeit der Prothesenhand zu messen.

4. Prothesenhand nach einem der vorangehenden Ansprüche, wobei das erste Radarmodul (5) und/oder das zweite Radarmodul (6) ein frequenzmoduliertes Dauerstrichradar (Frequency Modulated Continuous Wave, FMCW) ist.

5. Prothesenhand nach einem der vorangehenden Ansprüche, wobei das erste Radarmodul (5) ein MIMO-Radar (Multiple Input, Multiple Output) ist.

6. Prothesenhand nach einem der vorangehenden Ansprüche, wobei das erste Radarmodul (5), das zweite Radarmodul (6) und die Verarbeitungseinheit in einem Hardware-Regelkreis miteinander verbunden sind, in dem das erste Radarmodul (5) Teil eines MIMO-Radarmoduls (104) ist, das zweite Radarmodul (6) Teil eines SISO-Radarmoduls (102) ist und die Verarbeitungseinheit Teil einer Prothesenhand-Steuereinheit ist, wobei das MIMO-Radarmodul (104), das SISO-Radarmodul (102) und die Prothesenhand-Steuereinheit jeweils in mindestens einem Mikrocontroller implementiert sind.

7. Prothesenhand nach einem der vorangehenden Ansprüche, wobei das MIMO-Radarmodul (104) eine Digitalsignalverarbeitungseinheit (DSP) (108) umfasst, die eingerichtet ist, einen adaptiven Filterprozess, eine schnelle Fourier-Transformation, durchzuführen und/oder eingerichtet ist, andere Einheiten des MIMO-Radarmoduls (104) zu steuern, und/oder eine Funkfrequenz-Untereinheit, die eingerichtet ist, Sende- und Empfangsantennen des MIMO-Radarmoduls (104) zu parametrisieren und/oder eine Bandbreite und/oder Chirp-Dauer anzupassen.

8. Verfahren zum Bedienen einer Prothesenhand, wobei die Prothesenhand eine Prothesenhand nach einem der vorangehenden Ansprüche ist, wobei in einer Transportphase das erste Radarmodul (5) das von dem Zielobjekt (7) reflektierte erste Radarsignal empfängt und sich das Handgelenk (2) auf das Zielobjekt (7) zu bewegt, und in einer Greifphase das zweite Radarmodul (6) das von dem Zielobjekt (7) reflektierte zweite Radarsignal empfängt und sich der mindestens eine Finger (3) zum Greifen des Zielobjekts (7) formt,
wobei mindestens eine Eigenschaft auf der Grundlage des ersten Eigenschaftssignals bestimmt wird, das von dem ersten Radarmodul (5) auf der Grundlage des empfangenen ersten Radarsignals erzeugt wird, und
wobei aus dem von dem zweiten Radarmodul (6) erzeugten zweiten Eigenschaftssignal auf der Grundlage des zweiten Radarsignals ein Material des Zielobjekts (7) bestimmt wird.

9. Verfahren nach einem der vorangehenden Ansprüche, wobei während der Transportphase eine primäre Form des Zielobjekts (7), eine Größe des Zielobjekts (7), eine Höhe des Zielobjekts (7), eine Breite des Zielobjekts (7) und/oder scharfe Kanten des Zielobjekts (7) erfasst werden.

10. Verfahren nach einem der Ansprüche 8 oder 9, wobei anhand eines Winkelbewegungsmusters der Prothesenhand erfasst wird, dass sich die Prothesenhand in der Transportphase befindet.

11. Verfahren nach einem der Ansprüche 8 bis 10, wobei das erste Radarmodul (5) einen Abtastprozess durchführt, bei dem eine zweidimensionale Karte des Zielobjekts (7) in einer Ebene, die das Zielobjekt (7) schneidet, festgehalten wird.

12. Verfahren nach einem der vorangehenden Ansprüche, wobei ein Kippwinkel erworben wird, wobei auf der Grundlage des Kippwinkels die zweidimensionale Karte korrigiert wird.

13. Verfahren nach einem der zwei vorangehenden Ansprüche, wobei eine primäre Form des Zielobjekts (7), eine Größe des Zielobjekts (7), eine Höhe des Zielobjekts (7), eine Breite des Zielobjekts (7), scharfe Kanten des Zielobjekts (7), eine Position des Zielobjekts (7) und/oder eine Orientierung des Zielobjekts (7) durch adaptive Filterung der Ergebnisse des Abtastprozesses berechnet wird.

14. Verfahren nach einem der Ansprüche 8 bis 12, wobei ein Material des Zielobjekts (7) auf der Grundlage von Änderungen in dem zweiten Radarsignal, das von dem zweiten Radarmodul (6) aufgezeichnet wird, erfasst wird.

15. Verfahren nach einem der Ansprüche 8 bis 13, wobei Störungen zwischen dem ersten (5) und/oder zweiten Radarmodul (6) und dem Zielobjekt (7) durch den Einsatz einer Maschinen-Lerntechnik und/oder einer Tiefenlern-Technik, vorzugsweise unter Verwendung eines Generative Adversarial Network (GAN), kompensiert werden.

## Revendications

1. Prothèse de main comprenant,
une articulation du poignet (2),
au moins un doigt (3),
un premier module radar (5) fixé à la prothèse de main au niveau de l'articulation du poignet (2), le premier module radar (5) étant configuré pour recevoir un premier signal radar réfléchi par un objet cible (7) et générer un premier signal de propriété sur la base du premier signal radar reçu,
un second module radar (6) fixé au doigt, le second module radar (6) étant configuré pour recevoir un second signal radar réfléchi par l'objet cible (7) et générer un second signal de propriété sur la base du second signal radar reçu,
une unité de traitement configurée pour recevoir les premier et second signaux de propriété et déterminer au moins une propriété de l'objet cible (7) sur la base du premier signal de propriété et déterminer un matériau de l'objet cible (7) sur la base du second signal de propriété.

2. Prothèse de main selon la revendication précédente, dans laquelle l'unité de traitement est configurée pour détecter sur la base du premier signal de propriété une forme principale de l'objet cible (7), une taille de l'objet cible (7), une hauteur de l'objet cible (7), une largeur de l'objet cible (7) et/ou des arêtes vives de l'objet cible (7).

3. Prothèse de main selon l'une quelconque des revendications précédentes, dans laquelle la prothèse de main comprend en outre au moins un capteur, de préférence une unité de mesure inertielle, configurée pour mesurer un angle d'inclinaison et/ou une vitesse angulaire de la prothèse de main.

4. Prothèse de main selon l'une quelconque des revendications précédentes, dans laquelle le premier module radar (5) et/ou le second module radar (6) est un radar à onde continue modulée en fréquence, FMCW.

5. Prothèse de main selon l'une quelconque des revendications précédentes, dans laquelle le premier module radar (5) est un radar à entrée multiple, sortie multiple MIMO (Multiple Input, Multiple Output).

6. Prothèse de main selon l'une quelconque des revendications précédentes, dans laquelle le premier module radar (5), le second module radar (6) et l'unité de traitement sont interconnectés dans une boucle de commande matérielle dans laquelle le premier module radar (5) fait partie d'un module radar MIMO (104), le second module radar (6) fait partie d'un module radar SISO (102) et l'unité de traitement fait partie d'une unité de commande de prothèse de main, dans lequel le module radar MIMO (104), le module radar SISO (102) et l'unité de commande de prothèse de main sont chacun mis en oeuvre dans au moins un microcontrôleur.

7. Prothèse de main selon la revendication précédente, dans laquelle le module radar MIMO (104) comprend une unité de traitement de signal numérique (DSP) (108), configurée pour réaliser un processus de filtrage adaptatif, une transformée de Fourier rapide et/ou configurée pour commander d'autres unités du module radar MIMO (104), et/ou une sous-unité à radiofréquence, configurée pour paramétrer les antennes d'émission et de réception du module radar MIMO (104) et/ou ajuster une bande passante et/ou une durée de compression d'impulsions.

8. Procédé de fonctionnement d'une prothèse de main, la prothèse de main étant une prothèse de main selon l'une des revendications précédentes, dans lequel dans une phase de transport, le premier module radar (5) reçoit le premier signal radar réfléchi par l'objet cible (7) et le poignet (2) se déplace vers l'objet cible (7) et dans une phase de saisie, le second module radar (6) reçoit le second signal radar réfléchi par l'objet cible (7) et le au moins un doigt (3) se forme pour saisir l'objet cible (7),
dans lequel au moins une propriété est déterminée sur la base du premier signal de propriété généré par le premier module radar (5) en fonction du premier signal radar reçu et
dans lequel un matériau de l'objet cible (7) est déterminé à partir du second signal de propriété généré par le second module radar (6) sur la base du second signal radar.

9. Procédé selon la revendication précédente, dans lequel pendant la phase de transport, une forme principale de l'objet cible (7), une taille de l'objet cible (7), une hauteur de l'objet cible (7), une largeur de l'objet cible (7) et/ou des arêtes vives de l'objet cible (7) sont détectées.

10. Procédé selon l'une des revendications 8 ou 9, dans lequel il est détecté que la prothèse de main est en phase de transport à partir d'un modèle de mouvement angulaire de la prothèse de main.

11. Procédé selon l'une des revendications 8 à 10, dans lequel le premier module radar (5) exécute un processus de balayage dans lequel une carte bidimensionnelle de l'objet cible (7) dans un plan coupant l'objet cible (7) est capturée.

12. Procédé selon la revendication précédente dans lequel un angle d'inclinaison est acquis, dans lequel, sur la base de l'angle d'inclinaison, la carte bidimensionnelle est corrigée.

13. Procédé selon l'une des deux revendications précédentes, dans lequel une forme principale de l'objet cible (7), une taille de l'objet cible (7), une hauteur de l'objet cible (7), une largeur de l'objet cible (7), des arêtes vives de l'objet cible (7), une position de l'objet cible (7) et/ou une orientation de l'objet cible (7) sont calculées par un filtrage adaptatif des résultats du processus de balayage.

14. Procédé selon l'une des revendications 8 à 12, dans lequel un matériau de l'objet cible (7) est détecté en fonction des variations du second signal radar enregistré par le second module radar (6).

15. Procédé selon l'une des revendications 8 à 13, dans lequel les perturbations entre le premier (5) et/ou le second module radar (6) et l'objet cible (7) sont compensées en employant une technique d'apprentissage automatique et/ou une technique d'apprentissage profond, de préférence en utilisant un réseau antagoniste génératif.
